# EUROPÄISCHE PATENTSCHRIFT

(11) **EP 0 891 894 B1**
(45) Veröffentlichungstag und Bekanntmachung des Hinweises auf die Patenterteilung: **14.09.2005**
(21) Anmeldenummer: 98112529.7
(22) Anmeldetag: 07.07.1998
(51) Int. Cl.: B60R 16/02

(54) **Verfahren zur Bewertung von Betätigungen des Fahrpedales durch einen Fahrzeugführer zur Erkennung der Aktivität und Nervosität des Fahrzeugführers**
Procedure for evaluating the driver's actuatings of the accelerator pedal for recognizing the driver's activity and nervosity
Procédé d'évaluation des actionnements de la pédale d'accélérateur par un conducteur de véhicule pour la reconnaissance de l'activité et de la nervosité de ce dernier

(30) Priorität: 18.07.1997 DE 19730904
(43) Veröffentlichungstag der Anmeldung: 20.01.1999
(73) Patentinhaber: DaimlerChrysler AG, 70567 Stuttgart (DE)
(72) Erfinder: Kuhn, Klaus-Peter, Dr., 73655 Plüderhausen (DE); Strenkert, Jochen, Dr., 70569 Stuttgart (DE)

(56) Entgegenhaltungen:
- EP-A- 0 282 387
- FR-A- 2 697 884

## Beschreibung

Die vorliegende Erfindung betrifft ein Verfahren zur Bewertung von Betätigungen des Fahrpedales durch einen Fahrzeugführer zur Erkennung der Nervosität des Fahrzeugführers nach dem Oberbegriff des Patentanspruches 1.

Ein derartiges Verfahren ist aus FR 2 697 884 bekannt.

Weiterhin sind verschiedene Verfahren bekannt, nach dem die Art und Weise der Betätigung des Fahrpedals durch den Fahrzeugführer ausgewertet wird, um die Fahrweise zu unterscheiden zwischen einer ruhigen und einer sportlichen Fahrweise. Bei dieser Unterscheidung sind selbstverständlich auch Zwischenschritte möglich bis hin zu einer kontinuierlichen Einstufung der Fahrweise. Derartige Verfahren sind beispielsweise beschrieben in dem Magazin Forschung und Technik der VW AG: Fuzzy-Logik für Automatikgetriebe, 1994. Weiterhin ist ein solches Verfahren beschrieben in der Automobiltechnischen Zeitschrift Nr. 97, 1995 von Rösch, R. und Wagner, G.: Die eletronische Steuerung des automatischen Getriebes W5A 330/580 von Mercedes-Benz. Bei diesen Verfahren kann es unter Umständen vorkommen, daß nervöse Fahrzeugführer als Fahrzeugführer mit einer sportlichen Fahrweise eingestuft werden. Das Fahrzeug kann dann eventuell ein für diesen Fahrzeugführer nicht optimales Fahrverhalten aufweisen.

Aus der DE 44 01 416 A1 ist es bekannt, eine Bewertung einer Fahrweise erst dann vorzunehmen, wenn innerhalb eines Zeitfensters eine signifikante Geschwindigkeitsänderung eingetreten ist, die allerdings auch nicht zu groß sein darf. Dadurch können lediglich auf einer Nervosität des Fahrzeugführers beruhende Aktionen bei der Auswertung und Bewertung der Fahrweise ausgeklammert werden. Indem die Geschwindigkeitsänderung nicht zu groß sein darf, wird weiterhin berücksichtigt, daß es sich mutmaßlich um eine Geschwindigkeitsänderung handelt, die der Fahrzeugführer "aus freien Stücken" vorgibt und die im nicht aufgrund der Verkehrsverhältnisse aufgezwungen wurde.

Weiterhin ist es aus der DE 43 37 957 A1 bekannt, die Häufigkeit eines Richtungswechsels der Verstellung einer Drosselklappe und damit auch die Häufigkeit eines Richtungswechsels bei der Betätigung des Fahrpedals eines Kraftfahrzeuges auszuwerten, um eine eventuelle Nervosität des Fahrzeugführers zu erkennen.

Es ist Aufgabe der vorliegenden Erfindung, ein Verfahren vorzuschlagen, mit dem eine Nervosität eines Fahrzeugführers erkannt werden kann.

Bei dem erfindungsgemäßen Verfahren nach Anspruch 1 wird in einem vorgegebenen Zeitfenster der betragsmäßig kumulierte zurückgelegte Weg des Fahrpedals ausgewertet, wenn in diesem Zeitfenster die Geschwindigkeitsänderung kleiner ist als ein bestimmter Schwellwert und/oder die Änderung der Stellung des Fahrpedales kleiner ist als ein bestimmter Schwellwert.

Während also beim Stand der Technik eine Erkennung der Fahrweise erst dann möglich war, wenn aufgrund der Fahrsituation geschlossen werden konnte, daß die Aktion des Fahrzeugführers nicht mehr auf einer nervösen Aktion beruhte, kann mit dem erfindungsgemäßen Verfahren die Nervosität des Fahrzeugführers bereits vorab erkannt werden. Bei anderen Verfahren wie beispielsweise einem Eingriff in ein Motor-/Getriebemanagement eines Fahrzeuges in Abhängigkeit einer ruhigen oder sportlichen Fahrweise wie beispielsweise auch in Abhängigkeit einer spontanen Dynamikanforderung kann also frühzeitig berücksichtigt werden, ob es sich um einen nervösen Fahrzeugführer handelt. Die Aktionen zur Erkennung der Fahrweise oder der Erkennung einer spontanen Dynamikanforderung können also frühzeitig entsprechend eingeordnet werden.

Bei der Ausgestaltung des Verfahrens nach Anspruch 2 wird die momentane Aktivität des Fahrzeugführers gebildet aus der gewichteten Summe des Kriteriums nach Anspruch 1 und einem weiteren Kriterium das durch eine Auswertung gebildet wird, wie oft sich das Vorzeichen der zeitlichen Ableitung der Stellung des Fahrpedals ändert, wobei aus dieser momentanen Aktivität weiter der Grad der Aktivität des Fahrzeugführers durch die je nach Vorzeichen der Änderung der momentanen Aktivität unterschiedlich gefilterten, normierten momentanen Aktivität gebildet wird.

Dadurch können besonders vorteilhaft schnelle Änderungen der Stellung des Fahrpedales erkannt werden. Aufgrund dessen kann dann eine Einordnung in Bezug zu einer eventuell vorliegenden Nervosität erfolgen.

Diese Kennzahl für den Grad der Aktivität kann beispielsweise bei der Adaption der Schwellwerte zur Erkennung eines dynamischen Kickdowns besonders vorteilhaft ausgewertet werden.

Bei dem Verfahren nach Anspruch 3 wird anhand eines der Verfahren nach einem der vorgenannten Ansprüche die Aktivität des Fahrzeugführers ermittelt und weiterhin die Fahrweise bewertet als zwischen ruhig und sportlich liegend. Eine Nervosität des Fahrzeugführers wird erkannt, wenn die Aktivität groß ist und die Fahrweise nicht als sportlich bewertet wird.

Dadurch kann besonders vorteilhaft ausgewertet werden, ob eine Aktivität des Fahrzeugführers zielgerichtet ist und sich auch in entsprechenden Beschleunigungen des Fahrzeuges niederschlägt. Dadurch kann besonders sicher ein Fahrzeugführer mit einer sportlichen Fahrweise von einem nervösen Fahrzeugführer unterschieden werden.

Bei dem Verfahren nach Anspruch 4 wird die Nervosität des Fahrzeugführers nur ermittelt, wenn die Fahrweise nicht als sportlich bewertet wird.

Dadurch kann weiterhin vorteilhaft eine Einordnung eines Fahrzeugführers mit einer sportlichen Fahrweise als nervöser Fahrzeugführer unterbunden werden. Dies ist besonders dann vorteilhaft, wenn die Bewertung der Fahrweise als ruhig oder sportlich nicht durch die Auswertung der Betätigung beispielsweise des Fahrpedales erfolgt sondern durch das sich einstellende Fahrverhalten des Fahrzeuges wie dies beispielsweise in der DE 44 01 416 A1 beschrieben ist Die beiden Kriterien Nervosität des Fahrzeugführers einerseits und Fahrweise des Fahrzeugführers andererseits werden dann aus unterschiedlichen Daten (Betätigung des Fahrpedales einerseits und erzielte Beschleunigung andererseits) gewonnen, so daß die beiden zu untersuchenden Kriterien gut trennbar sind. Andernfalls kann es vorkommen, daß eine Erkennung der Nervosität unterbunden werden soll, wenn eine sportliche Fahrweise vorliegt, wobei dann aber die Einordnung schwierig wird, ob die Betätigung des Fahrpedales nun auf einer sportlichen Fahrweise oder auf der Nervosität des Fahrzeugführers beruht.

Bei dem Verfahren nach Anspruch 5 wird ein Bit gesetzt bei einer erkannten Nervosität und das Bit gelöscht, wenn die Bedingungen für die Erkennung der Nervosität nicht mehr vorliegen und die Stellung des Fahrpedales gleich 0 ist.

Durch dieses scharfe Kriterium der Erkennung der Nervosität können dann gegebenenfalls bestimmte Eingriffe in das Motor-/Getriebemanagement unterbunden werden, wenn eine Nervosität erkannt wurde. Dies ist im einzelnen in den beiden Patentanmeldungen der Anmelderin beschrieben, die am selben Anmeldetag eingereicht worden sind und die amtlichen Aktenzeichen des deutschen Patentamtes 197 30 906.2 sowie 197 29 251.8.

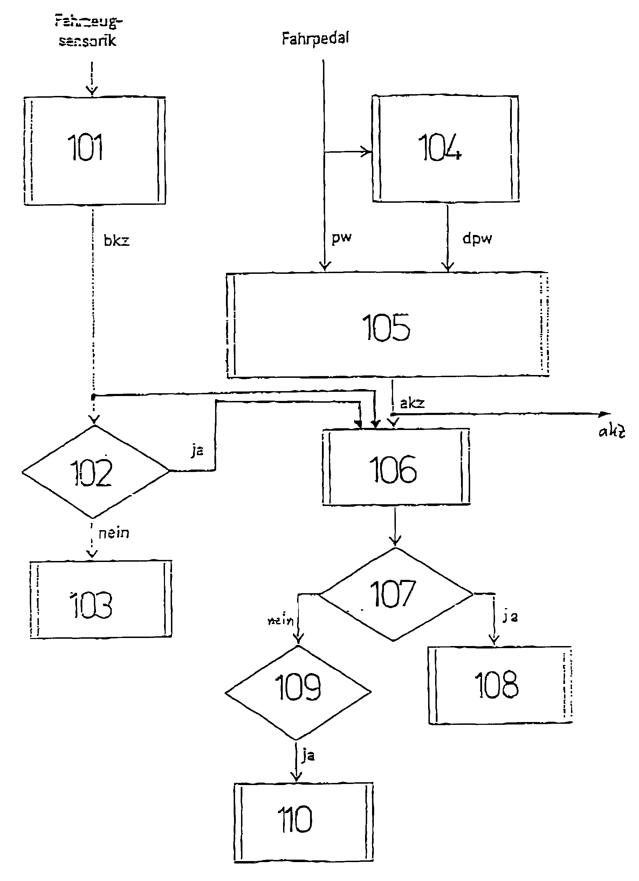

Ein Ausführungsbeispiel ist in der Figur näher dargestellt. In dem Block 101 wird die Fahrweise des Fahrzeugführers als ruhig bzw. als sportlich bewertet. Dies kann beispielsweise nach dem in der DE 44 01 416 A1 beschriebenen Verfahren erfolgen. Vorteilhaft wird in dem hier vorliegenden Fall die Beschleunigungskennziffer bkz verwendet.

In dem Block 102 wird überprüft, ob diese Beschleunigungskennziffer bkz kleiner ist als ein vorgegebener Schwellwert.

Wenn dies nicht der Fall ist und der Fahrzeugführer also eine eher sportliche Fahrweise hat, erfolgt ein Übergang zu dem Block 103, in dem das Nervositätsbit nb auf 0 gesetzt wird.

Wurde in dem Block 102 festgestellt, daß die Beschleunigungskennziffer kleiner ist als der Schwellwert, so erfolgt ein Übergang zu dem Block 106. In Block 106 wird das Kriterium gebildet, anhand dessen die Nervosität erkannt werden soll. Dazu wird, falls die Bedingung aus Block 102 erfüllt ist, eine Maßzahl für die Korrelation aus Beschleunigungskennziffer bkz und Aktivitätsgrad akz gebildet.

Der Aktivitätsgrad akz wird im Block 105 ermittelt. Diesem Block wird ein die Stellung des Fahrpedales repräsentierendes Signal pw sowie ein die zeitliche Ableitung dieses Signales repräsentierendes Signal dpw zugeführt. Die zeitliche Ableitung wird in dem Block 104 gewonnen.

In dem Block 105 werden die Kriterien für die Erkennung einer Nervosität des Fahrzeugführers gebildet. Das erste Kriterium errechnet sich aus dem betragsmäßig kumulierten Weg den das Fahrpedal in einem Zeitfenster zurückgelegt hat. In diesem Zeitfenster darf dabei keine signifikante Geschwindigkeitsänderung und/oder Änderung der Stellung des Fahrpedales vorkommen. Damit ist eine Änderung der Stellung des Fahrpedales gemeint, die bei einer Bewegung ohne Bewegungsumkehr einen bestimmten Betrag überschreitet. Der kumulierte Weg hingegen wird gerade bei einem als nervös einzustufenden Fahrzeugführer vergleichsweise groß ausfallen. Der kumulierte Weg wird selbstverständlich vorteilhafterweise um das Signalrauschen korrigiert.

In dem Block 105 wird ein weiteres Kritierum gebildet anhand der Anzahl der Vorzeichenwechsel der zeitlichen Änderung der Stellung des Fahrpedales in dem betrachteten Zeitfenster.

Ebenfalls in Block 105 wird aus diesen beiden Kriterien die momentane Aktivität als gewichtete Summe der normierten Kennzahlen ermittelt. Der Grad der Aktivität akz errechnet sich aus der je nach Vorzeichen der Änderung unterschiedliche gefilterten normierten momentanen Aktivität Die Aktivitätszahl steht, ähnlich der Beschleunigungskennziffer bkz, als Kriterium beispielsweise für Eingriffe in das Motor-/Getriebemanagament zur Verfügung.

In dem Block 107 wird das in dem Block 106 gebildetet Kriterium daraufhin untersucht, ob dies eine bestimmte Schwelle überschreitet.

Ist dies der Fall, erfolgt ein Übergang zu dem Block 108, in dem das Nervositätsbit nb auf 1 gesetzt wird.

Ist dies nicht der Fall, erfolgt ein Übergang zu dem Block 109, in dem weiter geprüft wird, ob das Fahrpedal auf 0 steht. Ist dies der Fall, wird in dem Schritt 110 das Nervositätsbit nb auf 0 gesetzt.

Im Ergebnis ergibt sich also ein Nervositätsbit nb, das eine scharfe Trennung repräsentiert, ob der Fahrzeugführer nervös ist (ja/nein). Zusätzlich ist eine weitere Kenngröße verfügbar, die den Grad der individuellen Aktivität akz widerspiegelt. Diese Informationen können beispielsweise bei der Adaption von Fahrpedalcharakteristiken und/oder Getriebeschaltlinien dazu verwendet werden, auch einem nervösen Fahrzeugführer durch eine entsprechend ruhige Fahrstrategie größtmöglichen Fahrkomfort und gutes Fahrzeughandling bei gutem Verbrauch zu ermöglichen. Dasselbe gilt auch beispielsweise für die Zuordnung Fahrpedal / Drosselklappe bzw. Einspritzmenge, die durch eine entsprechende Signalverarbeitung indirekter gestaltet werden kann, beispielsweise durch eine geeignete Tiefpaßfilterung.

## Patentansprüche

1. Verfahren zur Bewertung von Betätigungen des Fahrpedales durch einen Fahrzeugführer zur Erkennung der Nervosität des Fahrzeugführers bei dem eine Geschwindigkeitsänderung und/oder die Änderung der Stellung des Fahrpedals erfasst wird,
**dadurch gekennzeichnet, daß** in einem vorgegebenen Zeitfenster der betragsmäßig kumulierte zurückgelegte Weg des Fahrpedals ausgewertet wird, wenn in diesem Zeitfenster die Geschwindigkeitsänderung kleiner ist als ein bestimmter Schwellwert und/oder die Änderung der Stellung des Fahrpedales kleiner ist als ein bestimmter Schwellwert (105).

2. Verfahren nach Anspruch 1,
**dadurch gekennzeichnet daß** die momentane Aktivität des Fahrzeugführers gebildet wird aus der gewichteten Summe des Kriteriums nach Anspruch 1 und eines weiteren Kriteriums, das durch eine Auswertung gebildet wird, wie oft sich das Vorzeichen der zeitlichen Ableitung der Stellung des Fahrpedals ändert, und daß daraus weiter der Grad der Aktivität des Fahrzeugführers durch die je nach Vorzeichen der Änderung der momentanen Aktivität unterschiedlich gefilterten, normierten momentanen Aktivität gebildet wird (103).

3. Verfahren nach einem der Ansprüche 1 oder 2,
**dadurch gekennzeichnet daß** die Aktivität des Fahrzeugführers ermittelt wird und daß weiterhin die Fahrweise bewertet wird als zwischen ruhig und sportlich liegend und daß eine Nervosität des Famzeugführers erkannt wird, wenn die Aktivität groß ist und die Fahrweise nicht als sportlich bewertet wird (102, 106, 107).

4. Verfahren nach Anspruch 3,
**dadurch gekennzeichnet, daß** die Nervosität des Fahrzeugführers nur ermittelt wird, wenn die Fahrweise nicht als sportlich bewertet wird (102).

5. Verfahren nach einem der Ansprüche 1 bis 4,
**dadurch gekennzeichnet, daß** ein Bit gesetzt wird bei einer erkannten Nervosität und daß das Bit gelöscht wird, wenn die Bedingungen für die Erkennung der Nervosität nicht mehr vorliegen und die Stellung des Fahrpedales gleich 0 ist (103, 108, 110).

## Claims

1. Method of evaluating actuation of the accelerator pedal by a vehicle driver with a view to detecting the nervousness of the vehicle driver, whereby a speed change and/or the change in the position of the accelerator pedal is detected,
**characterised in that** the amount of cumulated travel of the accelerator pedal within in a pre-set time window is evaluated if the speed change within this time window is lower than a specific threshold value and/or the change in the position of the accelerator pedal is below a specific threshold value (105).

2. Method as claimed in claim 1,
**characterised in that** the instantaneous activity of the vehicle driver is derived on the basis of the weighted sum of the criterion specified in claim 1 and another criterion derived on the basis of an evaluation as to how often the algebraic sign of the time derivation of the position of the accelerator pedal changes, and this instantaneous activity is also used to derive the degree of activity of the vehicle driver on the basis of the normalised instantaneous activity, which is filtered differently depending on the algebraic sign of the change in instantaneous activity (105).

3. Method as claimed in one of claims 1 or 2,
**characterised in that** the activity of the vehicle driver is determined and the driving style is also evaluated as being somewhere between steady and sporty and a nervousness of the vehicle driver is detected if the activity is high and the driving style is evaluated as not being sporty (102, 106, 107).

4. Method as claimed in claim 3,
**characterised in that** the nervousness of the vehicle driver is not determined unless the driving style is evaluated as not being sporty (102).

5. Method as claimed in one of claims 1 to 4,
**characterised in that** a bit is set if nervousness is detected and the bit is deleted when the condition for detecting nervousness no longer prevails and the position of the accelerator pedal is equal to 0 (103, 108, 110).

## Revendications

1. Procédé pour l'évaluation d'actionnements de la pédale d'accélérateur par un conducteur de véhicule pour l'identification de la nervosité du conducteur du véhicule, selon lequel on détecte une variation de la vitesse et/ou la variation de la position de la pédale d'accélérateur,
**caractérisé en ce qu'**on évalue, dans une fenêtre temporelle prédéterminée, le trajet parcouru, cumulé en valeur absolue, de la pédale d'accélérateur, lorsque dans cette fenêtre temporelle, la variation de la vitesse est inférieure à une valeur de seuil déterminée et/ou la variation de la position de la pédale d'accélérateur est inférieure à une valeur de seuil déterminée (105).

2. Procédé selon la revendication 1, **caractérisé en ce que** l'activité instantanée du conducteur du véhicule est formée à partir de la somme pondérée du critère selon la revendication 1 et d'un autre critère, qui est formé par une évaluation de la fréquence avec laquelle le signe de la dérivée dans le temps de la position de la pédale d'accélérateur varie, et qu'en outre à partir de là le degré d'activité du conducteur du véhicule est formé (105) par l'activité instantanée normalisée, filtrée différemment en fonction du signe de la variation de l'activité instantanée.

3. Procédé selon l'une des revendications 1 ou 2, **caractérisé en ce que** l'activité du conducteur du véhicule est déterminée et qu'en outre le mode de conduite est évalué comme se situant entre un mode de conduite calme et un mode de conduite sportive et qu'une nervosité du conducteur du véhicule est identifiée lorsque l'activité est intense et que le mode de conduite n'est pas évalué comme conduite sportive (102,106, 107).

4. Procédé selon la revendication 3, **caractérisé en ce que** la nervosité du conducteur du véhicule est déterminée uniquement lorsque le mode de conduite n'est pas évalué comme étant une conduite sportive (102).

5. Procédé selon l'une des revendications 1 à 4, **caractérisé en ce qu'**un bit est positionné dans le cas d'une nervosité identifiée et que le bit est effacé lorsque les conditions pour l'identification de la nervosité ne sont plus présentes et que la position de la pédale d'accélérateur est égale à 0 (103,108,110).
